# EUROPEAN PATENT APPLICATION

(11) **EP 4 613 174 A1**
(43) Date of publication of application: **10.09.2025**
(21) Application number: 23885529.0
(22) Date of filing: 18.10.2023
(51) Int. Cl.: A61B 1/12, A61B 1/00

(54) **ENDOSCOPE**

(30) Priority: 01.11.2022 JP 2022175798
(71) Applicant: HOYA CORPORATION, Shinjuku-ku Tokyo 160-8347 (JP)
(72) Inventor: WATANABE, Toshiki, Tokyo 160-8347 (JP)
(74) Representative: Lucke, Andreas
(86) International application number: PCT/JP2023/037614
(87) International publication number: WO 2024/095767

(57) **Abstract**

An endoscope (10) includes: a convex lens (132) provided protrusively on a distal end surface (131) of an insertion portion (14); and an air and water supply nozzle (140) provided protrusively on the distal end surface (131) and configured to inject a fluid to the convex lens (132). The air and water supply nozzle (140) has one or a plurality of injection ports (141). In a case where the air and water supply nozzle (140) has one injection port (141), the injection port (141) extends in an intersecting direction intersecting with a protruding direction of the convex lens (132), and at least one edge closer to a distal end of the air and water supply nozzle (140) among two edges of the injection ports (141) facing each other in the protruding direction has a convex shape in the protruding direction. In a case where the air and water supply nozzle (140) has a plurality of injection ports (141), the plurality of injection ports (141) are formed along the intersecting direction, and a tangent line in contact with each of the plurality of injection ports (141) on a distal end side of the air and water supply nozzle (140) is convexly curved in the protruding direction.

## Description

### Technical Field

The present invention relates to an endoscope including: a convex lens provided protrusively on a distal end surface of an insertion portion; and a nozzle configured to inject a fluid to the convex lens.

The present application claims priority based on Japanese Patent Application No. 2022-175798 filed on November 1, 2022, the entire contents of which are incorporated herein by reference.

### Background Art

Conventionally, in an endoscope, an observation optical system for imaging a subject is provided at a tip of an insertion portion to be inserted into a body. Dirt such as mucus, blood and residue is likely to adhere to a surface of such an observation optical system. As described above, if the observation optical system is dirty or the like, it is difficult to take a clear image of the subject.

In this regard, Patent Literature 1 discloses an endoscope that includes a convex observation window and a nozzle protruding from the observation window, and performs cleaning by injecting a fluid from the nozzle toward an apex of the observation window.

### Citation List

### Patent Literature

Patent Literature 1: JP 2012-120701 A

### Summary of Invention

### Technical Problem

However, in a case where the convex observation window is provided on a distal end surface of an insertion portion for wide-angle viewing, the nozzle needs to inject the fluid over a predetermined range in a protruding direction of the observation window. However, in a case where the fluid is injected toward the apex of the observation window, the fluid is difficult to reach an edge portion of the observation window, and cleaning may be insufficient. In addition, when the fluid is injected toward the edge portion of the observation window, the fluid collides with the observation window and is easily repelled, and there is a possibility that the fluid does not easily reach the apex of the observation window.

However, the endoscope of Patent Literature 1 does not devise such a problem and cannot solve the problem.

The present invention has been made in view of such circumstances, and an object of the present invention is to provide an endoscope capable of sufficiently and efficiently cleaning a convex lens provided on a distal end surface of an insertion portion.

### Solution to Problem

An endoscope according to the present invention includes: a convex lens provided protrusively on a distal end surface of an insertion portion; and a nozzle provided protrusively on the distal end surface and configured to inject a fluid to the convex lens. The nozzle has one or a plurality of injection ports. In a case where the nozzle has one injection port, the injection port extends in an intersecting direction intersecting with a protruding direction of the convex lens, and at least one edge close to a distal end of the nozzle among two edges of the injection port facing each other in the protruding direction has a convex shape in the projecting direction. In a case where the nozzle has a plurality of injection ports, the plurality of injection ports are formed along the intersecting direction, and a tangent line in contact with each of the plurality of injection ports on a distal end side of the nozzle is convexly curved in the protruding direction.

In the present invention, in a case where the nozzle has one injection port, at least one edge close to the distal end of the nozzle among two edges of the injection port facing each other in the protruding direction has a convex shape in the projecting direction, and in a case where the nozzle has a plurality of injection ports, a tangent line in contact with each of the plurality of injection ports on the distal end side of the nozzle is convexly curved in the protruding direction. Therefore, when the nozzle injects air or water, the air or water is injected according to a curved surface shape of a surface of the convex lens, and the convex lens can be sufficiently and efficiently cleaned.

### Advantageous Effects of Invention

According to the present invention, it is possible to provide an endoscope capable of sufficiently and efficiently cleaning a convex lens provided on a distal end surface of an insertion portion.

### Brief Description of Drawings

Fig. 1 is an external view of an endoscope according to a first embodiment of the present invention.
Fig. 2 is an external view of a distal end portion of the endoscope according to the first embodiment of the present invention.
Fig. 3 is a diagram illustrating an air and water supply nozzle of the endoscope according to the first embodiment of the present invention.
Fig. 4 is a cross-sectional view taken along line IV-IV in Fig. 3.
Fig. 5 is a cross-sectional view taken along line V-V of Fig. 4.
Fig. 6 is an exemplary view illustrating a case where the distal end surface is a flat surface in the endoscope according to the first embodiment of the present invention.
Fig. 7 is a schematic view schematically illustrating an air and water supply nozzle of an endoscope according to a second embodiment of the present invention.
Fig. 8 is a schematic view schematically illustrating an air and water supply nozzle of an endoscope according to a third embodiment of the present invention.
Fig. 9 is a schematic view schematically illustrating an air and water supply nozzle of an endoscope according to a fourth embodiment of the present invention.
Fig. 10 is a schematic view schematically illustrating a modification of the air and water supply nozzle of Fig. 9.
Fig. 11 is a schematic view schematically illustrating an air and water supply nozzle of an endoscope according to a fifth embodiment of the present invention.
Fig. 12 is a schematic view schematically illustrating a modification of the air and water supply nozzle of Fig. 11.
Fig. 13 is a schematic view schematically illustrating an air and water supply nozzle of an endoscope according to a sixth embodiment of the present invention.

### Description of Embodiments

Hereinafter, an endoscope according to embodiments of the present invention will be described in detail with reference to the drawings.

### (First Embodiment)

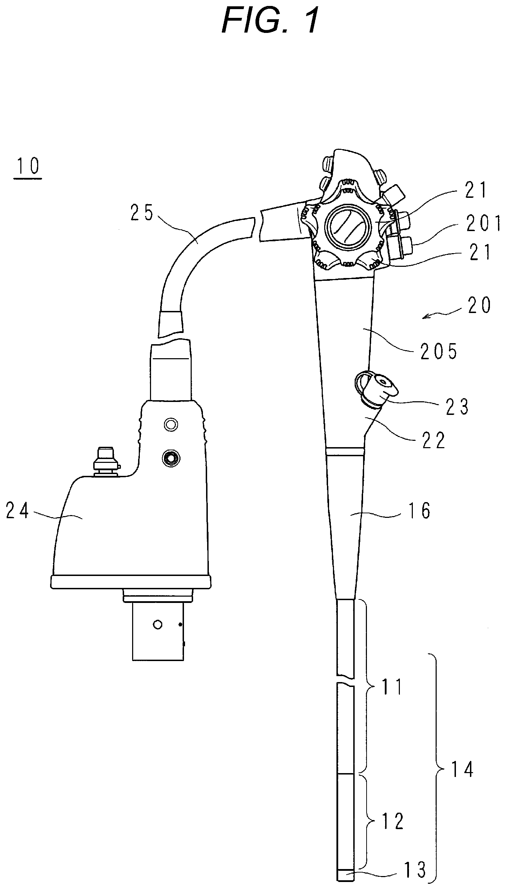

Fig. 1 is an external view of an endoscope 10 according to a first embodiment of the present invention. The endoscope 10 according to this embodiment includes an insertion portion 14, an operation unit 20, a universal cord 25, and a connector unit 24. The operation unit 20 includes a button 201 and a bending knob 21 for receiving a user operation, and a channel inlet 22 provided in a case 205 having a substantially cylindrical shape. A biopsy valve 23 having an insertion port for inserting a treatment tool or the like is attached to the channel inlet 22.

The insertion portion 14 is inserted into a body of a subject. The insertion portion 14 has an elongated cylindrical shape, and includes a distal end portion 13, a bending section 12, and a soft portion 11 in order from one end in the distal end. That is, the distal end portion 13, the bending section 12, and the soft portion 11 are positioned on the same axis. The other end of the insertion portion 14 is connected to the operation unit 20 via a bend preventing portion 16. The bending section 12 is curved according to an operation of the bending knob 21.

The universal cord 25 is long, and has one end connected to the operation unit 20 and the other end connected to the connector unit 24. The universal cord 25 is soft. The connector unit 24 is connected to a processor for an endoscope (not illustrated), a light source device, a display device, an air and water supply device, and the like. By appropriately operating the operation unit 20, a cleaning fluid (air or water) sent through the connector unit 24 is sent to the distal end portion 13 via the bend preventing portion 16.

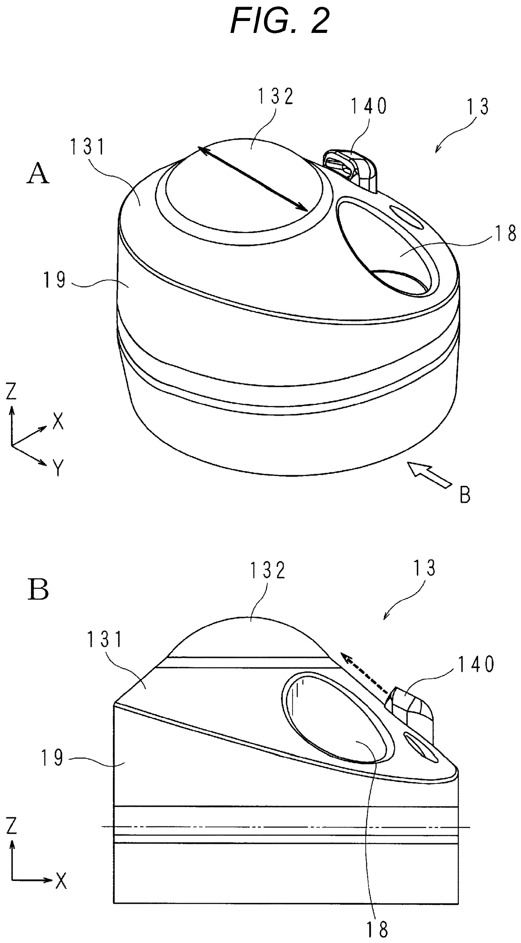

Fig. 2 is an external view of the distal end portion 13 of the endoscope 10 according to the first embodiment of the present invention. Fig. 2A is a perspective view of the distal end portion 13, and Fig. 2B is a view as seen in a direction of an arrow B. Hereinafter, an axial direction of the distal end portion 13 (insertion portion 14) is also referred to as a Z direction. Further, in Fig. 2B, a plane (XY plane) perpendicular to the Z direction is indicated by a two-dot chain line.

The distal end portion 13 has a substantially elliptical shape in an axial cross-sectional view, that is, in a cross-sectional view in the X direction or the Y direction, and the distal end thereof protrudes in the Z direction to form a substantially conical shape. A distal end surface 131 of the distal end portion 13 is provided with an objective lens 132 (convex lens), an air and water supply nozzle 140, a channel outlet 18, and the like.

Further, the distal end portion 13 has a cylindrical storage cylinder 19 which houses an image sensor (not illustrated) or the like that captures image light of the subject via the objective lens 132 and performs imaging. The distal end surface 131 of the distal end portion 13 is continuously provided from an edge of the storage cylinder 19. In the storage cylinder 19, the bending section 12, and the soft portion 11, a feeding path (not illustrated) of air or water injected to the objective lens 132 through the air and water supply nozzle 140 is formed.

The objective lens 132 is a hemispherical convex lens provided protrusively in the Z direction at a substantially central portion of the distal end surface 131 and supporting a wide viewing angle. Further, in the distal end surface 131, the air and water supply nozzle 140 and the channel outlet 18 are provided around the objective lens 132.

The distal end surface 131 of the distal end portion 13 looks like a truncated cone. The distal end surface 131 is an inclined surface extending from a circular edge portion of the objective lens 132 in a tangential direction thereof. The air and water supply nozzle 140 is attached to such an inclined surface (distal end surface 131), and the channel outlet 18 is formed. That is, the objective lens 132 is provided protrusively in the Z direction at the distal end of the distal end portion 13, the distal end surface 131 forms an inclined surface so as to surround the edge portion of the objective lens 132, and the air and water supply nozzle 140 and the channel outlet 18 are provided on the distal end surface 131 to be separated from the objective lens 132.

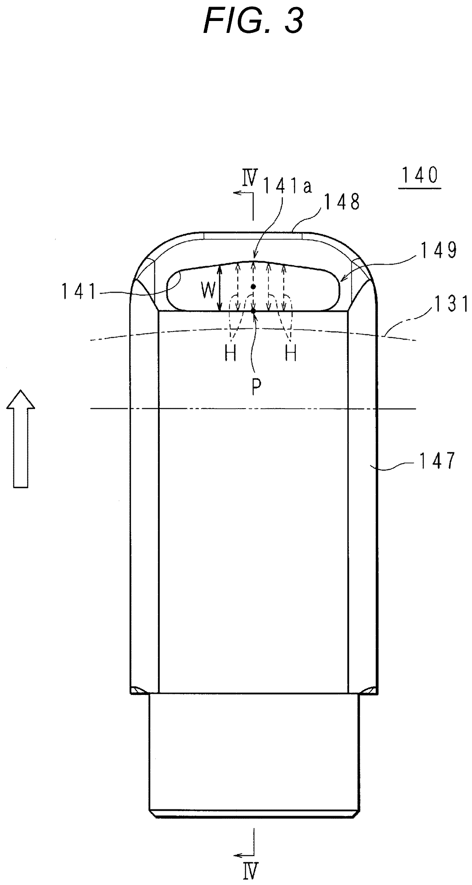

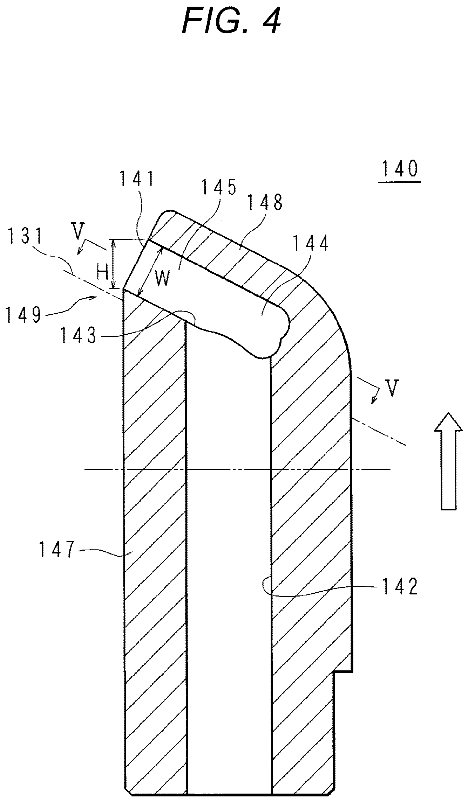

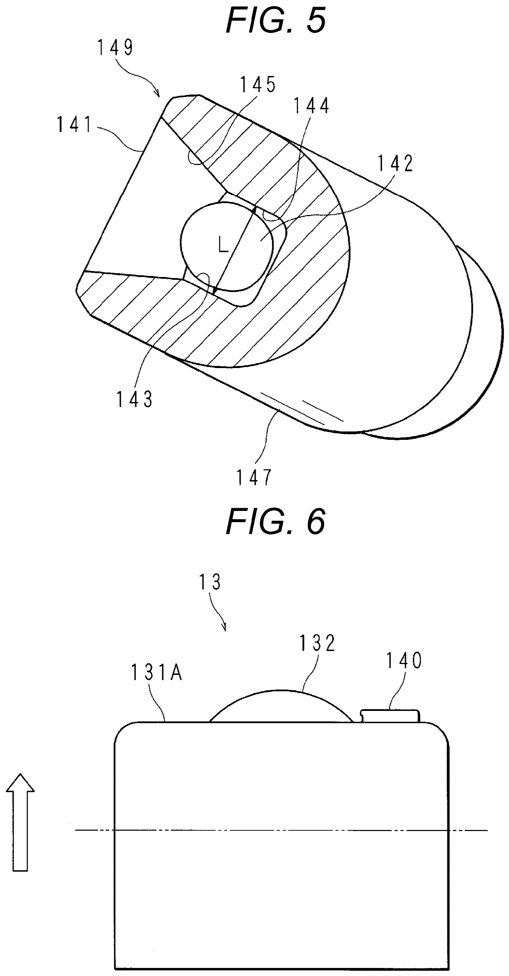

Fig. 3 is a view illustrating the air and water supply nozzle 140 of the endoscope 10 according to the first embodiment of the present invention, Fig. 4 is a cross-sectional view taken along line IV-IV of Fig. 3, and Fig. 5 is a cross-sectional view taken along line V-V of Fig. 4. In Figs. 3 and 4, the distal end surface 131 of the distal end portion 13 is indicated by a one-dot chain line for convenience of description. Further, in Figs. 3 and 4, the Z direction is indicated by an outlined arrow, and a plane (XY plane) perpendicular to the Z direction is indicated by a two-dot chain line.

The air and water supply nozzle 140 is inserted into and fixed to a recess formed on the distal end surface 131, on the operation unit 20 side with respect to the objective lens 132 (see Fig. 2). A distal end portion of the air and water supply nozzle 140 protrudes in the Z direction from the distal end surface 131. The air and water supply nozzle 140 injects air or water toward the objective lens 132 along the distal end surface 131 (see a dashed arrow in Fig. 2B). The air and water supply nozzle 140 injects air or water to the objective lens 132 over a predetermined range in an intersecting direction intersecting with the protruding direction of the objective lens 132, that is, an arrow direction (Y direction) in Fig. 2A. The air and water supply nozzle 140 injects air or water over a range corresponding to the objective lens 132 or a slightly wide range, in the intersecting direction.

As described above, since a viewing angle of the objective lens 132 is wide (180 degrees or more), in a case where the air and water supply nozzle 140 is arranged at the same position as the objective lens 132 in the axial direction of the insertion portion 14, the air and water supply nozzle 140 appears in a captured image obtained with the objective lens 132. However, in the endoscope 10 according to the first embodiment of the present invention, the air and water supply nozzle 140 is arranged on the operation unit 20 side with respect to the objective lens 132 as described above. Therefore, the air and water supply nozzle 140 does not appear in a captured image obtained with the objective lens 132, and does not interfere with imaging by the objective lens 132.

The air and water supply nozzle 140 includes: a cylindrical portion 147 through which air or water flows; a lid portion 148 which covers an open end on one side of the cylindrical portion 147; an injection portion 149 formed between the cylindrical portion 147 and the lid portion 148; and a first guide wall 144 and a second guide wall 145 which guide air or water from the cylindrical portion 147 to the injection portion 149. The lid portion 148, the injection portion 149, the cylindrical portion 147, the first guide wall 144, and the second guide wall 145 are integrally formed. Most of the air and water supply nozzle 140 except for the injection portion 149, the lid portion 148, the first guide wall 144, and the second guide wall 145 is inserted into the recess of the distal end surface 131. That is, similarly to the objective lens 132, the air and water supply nozzle 140 protrudes in the Z direction from the distal end surface 131.

The lid portion 148 is a deformed plate-shaped portion in which a central portion is thinner in the protruding direction than other portions when viewed from an opening 143 side described later, and is arranged obliquely with respect to a longitudinal direction of the cylindrical portion 147. The injection portion 149, the first guide wall 144, and the second guide wall 145 are provided at one end of the cylindrical portion 147 on the lid portion 148 side. The cylindrical portion 147 internally includes a connecting pipe portion 142 extending along the longitudinal direction of the cylindrical portion 147. The connecting pipe portion 142 has a circular shape in a cross-sectional view, and sends air or water sent via the connector unit 24 and the bend preventing portion 16 to the first guide wall 144, the second guide wall 145, and the injection portion 149. That is, the connecting pipe portion 142 communicates with the injection portion 149.

The injection portion 149 is a portion including an injection port 141 through which air or water is injected. Air or water from the connecting pipe portion 142 flows out from the opening 143 on the lid portion 148 side of the connecting pipe portion 142, is guided to the injection port 141 by the first guide wall 144 and the second guide wall 145, and is injected toward the objective lens 132 via the injection port 141.

When injecting air or water, the injection portion 149 injects the air or water so that a central portion in an intersecting direction of the injected air or water is higher than other portions, in a protruding direction (Z direction) of the air and water supply nozzle 140 or the objective lens 132 from the distal end surface 131 (or XY plane). In addition, hereinafter, the protruding direction of the air and water supply nozzle 140 is also simply referred to as a protruding direction.

An end surface of the cylindrical portion 147 on the opening 143 side is inclined corresponding to the distal end surface 131 (see Fig. 4), and the first guide wall 144 and the second guide wall 145 are provided on such an end surface of the cylindrical portion 147. The first guide wall 144 and the second guide wall 145 surround the opening 143, and only a part on the objective lens 132 side is opened. Further, the lid portion 148 covers the opening 143 of the cylindrical portion 147 with the first guide wall 144 and the second guide wall 145 interposed in between.

That is, a cavity is formed around the opening 143 of the cylindrical portion 147 by the first guide wall 144 and the second guide wall 145, the lid portion 148, and the end surface of the cylindrical portion 147 on the opening 143 side, and the injection port 141 is formed by the above-described open portions of the first guide wall 144 and the second guide wall 145, the lid portion 148, and the end surface of the cylindrical portion 147 on the opening 143 side.

The first guide wall 144 has a substantially U shape, and is provided at a position away from the opening 143 in a radial direction of the opening 143. Further, the second guide wall 145 is continuously provided to each of both end portions of the first guide wall 144 on the open side, and extends to the injection port 141. Inner walls of the first guide wall 144 and the second guide wall 145 intersect with an end surface of the cylindrical portion 147 on the opening 143 side. Two inner wall portions facing each other in the first guide wall 144 are spaced apart from the opening 143 by a constant distance in the radial direction.

Further, in a juxtaposition direction of the injection port 141 and the opening 143, an interval L (see Fig. 5) between the two inner wall portions of the first guide wall 144 facing each other is constant. However, the interval L in the second guide wall 145 increases toward the injection port 141. That is, the interval L expands from a boundary between the first guide wall 144 and the second guide wall 145 up to the injection port 141.

The injection port 141 is opened toward the objective lens 132, and has a substantially oval shape with the intersecting direction as a long axis direction. That is, the injection port 141 extends along the XY plane in a direction intersecting with the Z direction (see Fig. 3). In the injection port 141, one edge closer to a distal end of the air and water supply nozzle 140 among two edges facing in the protruding direction (Z direction) has a convex shape in the protruding direction, similarly to the objective lens 132. Whereas, another edge closer to the distal end surface 131 among the two edges is parallel to the XY plane (see Fig. 3).

Further, as illustrated in Fig. 3, an opening width W of the injection port 141 increases toward a central portion 141a in the intersecting direction. Here, as illustrated in Fig. 4, the opening width W indicates a dimension between the one edge and the another edge of the injection port 141.

In other words, in the Z direction perpendicular to the XY plane, a dimension H (see Fig. 4) from one point (see a point P in Fig. 3) of the another edge of the injection port 141 to a position corresponding to the one edge is large at the central portion 141a in the intersecting direction, as compared to other portions. That is, the injection port 141 is formed such that the dimension H increases toward the central portion 141a in the intersecting direction.

Further, in the injection port 141, round chamfering is performed on each of concave corners on both end sides of the one edge and concave corners on both end sides of the another edge in the intersecting direction. As a result, it is possible to prevent deterioration of a cleaning effect due to a decrease in injection pressure caused by turbulence in the fluid at the concave corner of the injection port 141.

With the configuration above, the air and water supply nozzle 140 of the endoscope 10 according to the first embodiment injects air or water according to a shape of the objective lens 132 which is a convex lens. That is, in the endoscope 10 according to the first embodiment, as described above, the opening width W of the injection port 141 in the protruding direction (Z direction) increases toward the central portion 141a, and the dimension H increases toward the central portion 141a.

Therefore, in the air and water supply nozzle 140 (injection port 141), when air or water is injected, the central portion in the intersecting direction of the injected air or water is higher than other portions, in the protruding direction (Z direction) from the distal end surface 131 (or XY plane). That is, air or water is injected according to a curved surface shape of a surface of the objective lens 132 which is a convex lens. Therefore, the objective lens 132 can be cleaned sufficiently and efficiently.

The Coanda effect is known in which a fluid flowing near a wall surface is attracted to the wall surface by the effect of fluid viscosity. Due to such a Coanda effect, air or water injected from the injection port 141 flows along a surface (curved surface) of the objective lens 132.

In addition, since the endoscope 10 according to the first embodiment uses the objective lens 132 of a convex lens to support a wide viewing angle as described above, an acquired captured image is in a state where the central portion is enlarged. That is, in the captured image, a percentage of a portion obtained with the central portion of the objective lens 132 is larger than a percentage of a portion obtained with a peripheral portion except for the central portion. Therefore, it is necessary to intensively clean the central portion of the objective lens 132.

In this regard, in the air and water supply nozzle 140 of the endoscope 10 according to the first embodiment, as described above, since the opening width W of the injection port 141 increases toward the central portion 141a, an injection amount of air or water increases in the central portion. Therefore, the central portion of the objective lens 132 can be intensively cleaned, and a better captured image can be acquired.

Whereas, air or water flowing out of the opening 143 spreads while being distributed over the first guide wall 144 and the second guide wall 145. However, a vortex of air or water having vectors in various directions is formed between the first guide wall 144 and the lid portion 148 and between the first guide wall 144 and an end surface of the cylindrical portion 147 on the opening 143 side due to a round surface (curved surface) generated unavoidably in manufacturing. When a vortex is formed, a loss occurs, which degrades detergency of air or water.

In this regard, in the endoscope 10 according to the first embodiment, as described above, the interval L between the two inner wall portions facing each other is constant in the first guide wall 144, but expands from the boundary between the first guide wall 144 and the second guide wall 145 up to the injection port 141. With such a configuration, in the endoscope 10 according to the first embodiment, air or water is straightened toward the objective lens 132 at the boundary between the first guide wall 144 and the second guide wall 145, and then the flow is distributed and spreads to the second guide wall 145. Therefore, it is possible to suppress formation of a vortex of air or water and to prevent degradation in detergency of air or water.

In the above description, a case has been described as an example in which the distal end surface 131 has an appearance of a substantially truncated cone, that is, a case where the distal end surface 131 is inclined with respect to the longitudinal direction of the insertion portion 14, but the present invention is not limited thereto.

Fig. 6 is an exemplary view illustrating a case where a distal end surface 131A is a flat surface in the endoscope 10 according to the first embodiment of the present invention. As illustrated in Fig. **6****,** even in a case where the distal end surface 131A is a flat surface, it is a matter of course that the air and water supply nozzle 140 exhibits the above-mentioned effect.

### (Second Embodiment)

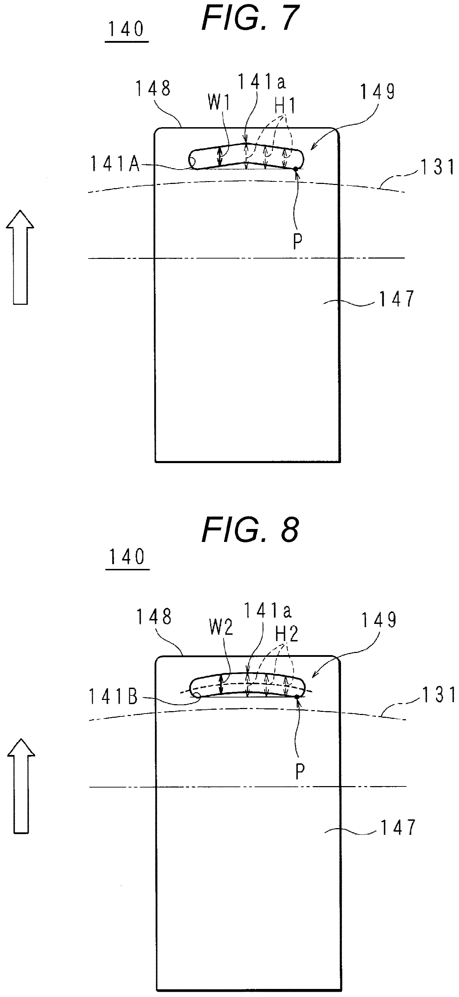

Fig. 7 is a schematic view schematically illustrating an air and water supply nozzle 140 of an endoscope 10 according to a second embodiment of the present invention. In Fig. 7, a distal end surface 131 of a distal end portion 13 is indicated by a one-dot chain line for convenience of description. Further, in Fig. 7, a Z direction is indicated by an outlined arrow, and a plane (XY plane) perpendicular to the Z direction is indicated by a two-dot chain line.

Similarly to the first embodiment, the air and water supply nozzle 140 includes a cylindrical portion 147, an injection portion 149, a lid portion 148, a first guide wall 144, and a second guide wall 145, and the lid portion 148, the injection portion 149, the cylindrical portion 147, the first guide wall 144, and the second guide wall 145 are integrally formed.

The injection portion 149 is a portion including an injection port 141A through which air or water is injected. When injecting air or water, the injection portion 149 injects the air or water so that a central portion in an intersecting direction of the injected air or water is higher than other portions, in a protruding direction (Z direction) of the air and water supply nozzle 140 (or an objective lens 132) from the distal end surface 131 (or XY plane).

The injection portion 149 of the second embodiment has a configuration similar to that of the first embodiment, but a shape of the injection port 141A is different.

The injection port 141A is opened toward the objective lens 132, has a substantially oval shape extending in the intersecting direction, and has an inverted V-shape. In the injection port 141A, one edge closer to a distal end of the air and water supply nozzle 140 among the two edges facing in the protruding direction (Z direction) has a convex shape in the protruding direction, similarly to the objective lens 132. Further, similarly to the one edge, another edge closer to the distal end surface 131 also has a convex shape in the protruding direction (see Fig. 7). An opening width W1 of the injection port 141A in the protruding direction is constant in the intersecting direction.

Further, in the injection port 141A, a dimension H1 (see a dashed arrow in Fig. 7) in the protruding direction (Z direction) from one point (see a point P in Fig. 7) of the another edge of the injection port 141A to a position corresponding to the another edge is large at a central portion 141a in the intersecting direction, as compared to other portions. More specifically, the injection port 141A is formed such that the dimension H1 increases toward the central portion 141a in the intersecting direction.

In the example of Fig. 7, a case where the one point (point P) is the position closest to the distal end surface 131 has been described as an example, but the present invention is not limited thereto.

With the above configuration, the air and water supply nozzle 140 of the endoscope 10 according to the second embodiment can inject air or water according to a curved surface shape of the objective lens 132 which is a convex lens.

That is, in the endoscope 10 according to the second embodiment, as described above, the injection port 141A is formed such that the dimension H1 is higher as being closer to the central portion 141a.

Therefore, in the air and water supply nozzle 140 (injection port 141A), when air or water is injected, the central portion in the intersecting direction of the injected air or water is higher than other portions, in the protruding direction (Z direction) from the distal end surface 131 (or XY plane). That is, air or water is injected according to a curved surface shape of a surface of the objective lens 132 which is a convex lens. Therefore, the objective lens 132 can be cleaned sufficiently and efficiently.

Parts similar to those in the first embodiment are denoted by the same reference numerals, and detailed descriptions thereof are omitted.

### (Third Embodiment)

Fig. 8 is a schematic view schematically illustrating an air and water supply nozzle 140 of an endoscope 10 according to a third embodiment of the present invention. In Fig. 8, a distal end surface 131 of a distal end portion 13 is indicated by a one-dot chain line for convenience of description. Further, in Fig. 8, a Z direction is indicated by an outlined arrow, and a plane (XY plane) perpendicular to the Z direction is indicated by a two-dot chain line.

Similarly to the first embodiment, the air and water supply nozzle 140 includes a cylindrical portion 147, an injection portion 149, a lid portion 148, a first guide wall 144, and a second guide wall 145, and the lid portion 148, the injection portion 149, the cylindrical portion 147, the first guide wall 144, and the second guide wall 145 are integrally formed.

The injection portion 149 is a portion including an injection port 141B through which air or water is injected. When injecting air or water, the injection portion 149 injects the air or water so that a central portion in an intersecting direction of the injected air or water is higher than other portions, in a protruding direction (Z direction) of the air and water supply nozzle 140 (or an objective lens 132) from the distal end surface 131 (or XY plane).

The injection portion 149 of the third embodiment has a configuration similar to that of the first embodiment, but a shape of the injection port 141B is different.

The injection port 141B is opened toward the objective lens 132, has a substantially oval shape extending in the intersecting direction, and has an arc shape. In the injection port 141B, one edge closer to a distal end of the air and water supply nozzle 140 among the two edges facing in the protruding direction (Z direction) has a convex shape in the protruding direction, similarly to the objective lens 132. Further, similarly to the one edge, another edge closer to the distal end surface 131 also has a convex shape in the protruding direction (see Fig. 8). An opening width W2 of the injection port 141B in the protruding direction is constant in the intersecting direction.

Further, in the injection port 141B, a dimension H2 (see a dashed arrow in Fig. 8) in the protruding direction (Z direction) from one point (see a point P in Fig. 8) of the another edge of the injection port 141B to a position corresponding to the another edge is large at a central portion 141a in the intersecting direction, as compared to other portions. More specifically, the injection port 141B is formed such that the dimension H2 increases toward the central portion 141a in the intersecting direction.

In the example of Fig. 8, a case where the one point (point P) is the position closest to the distal end surface 131 has been described as an example, but the present invention is not limited thereto.

With the above configuration, the air and water supply nozzle 140 of the endoscope 10 according to the third embodiment can inject air or water according to a curved surface shape of the objective lens 132 which is a convex lens.

That is, in the endoscope 10 according to the third embodiment, as described above, the injection port 141B is formed such that the dimension H2 is higher as being closer to the central portion 141a.

Therefore, in the air and water supply nozzle 140 (injection port 141B), when air or water is injected, the central portion in the intersecting direction of the injected air or water is higher than other portions, in the protruding direction (Z direction) from the distal end surface 131 (or XY plane). That is, air or water is injected according to a curved surface shape of a surface of the objective lens 132 which is a convex lens. Therefore, the objective lens 132 can be cleaned sufficiently and efficiently.

Parts similar to those in the first embodiment are denoted by the same reference numerals, and detailed descriptions thereof are omitted.

### (Fourth Embodiment)

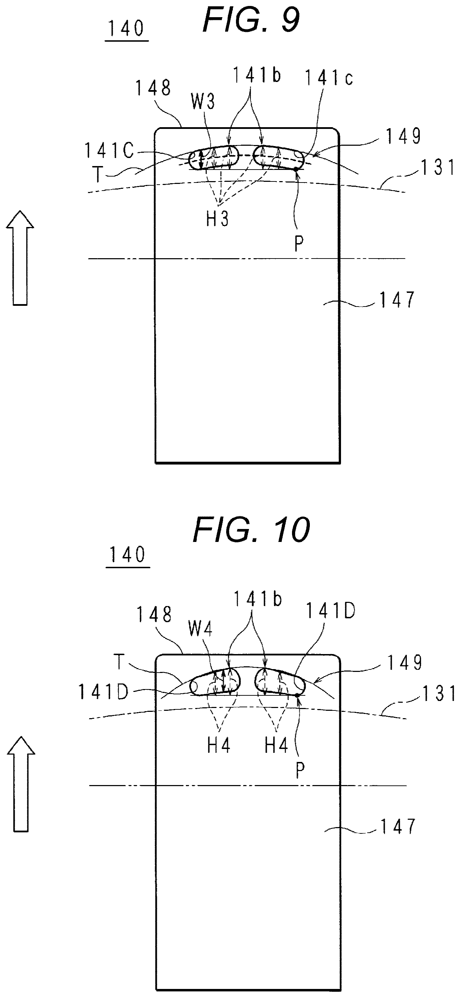

Fig. 9 is a schematic view schematically illustrating an air and water supply nozzle 140 of an endoscope 10 according to a fourth embodiment of the present invention. In Fig. 9, a distal end surface 131 of a distal end portion 13 is indicated by a one-dot chain line for convenience of description. Further, in Fig. 9, a Z direction is indicated by an outlined arrow, and a plane (XY plane) perpendicular to the Z direction is indicated by a two-dot chain line.

Similarly to the first embodiment, the air and water supply nozzle 140 includes a cylindrical portion 147, an injection portion 149, a lid portion 148, a first guide wall 144, and a second guide wall 145, and the lid portion 148, the injection portion 149, the cylindrical portion 147, the first guide wall 144, and the second guide wall 145 are integrally formed.

The injection portion 149 has two injection ports 141C through which air or water is injected, and is a portion including the two injection ports 141C and an interval between the two injection ports 141C.

When injecting air or water, the injection portion 149 injects the air or water so that a central portion in an intersecting direction of the injected air or water is higher than other portions, in a protruding direction (Z direction) of the air and water supply nozzle 140 (or an objective lens 132) from the distal end surface 131 (or XY plane). Further, the injection portion 149 has a configuration similar to that of the first embodiment, but is different in that the two injection ports 141C are included, and a shape of the injection port 141C is different.

The two injection ports 141C are juxtaposed in the intersecting direction. In addition, a tangent line T (see a solid line T in Fig. 9) in contact with each of the two injection ports 141C on the distal end side of the air and water supply nozzle 140 is convexly curved in the protruding direction, similarly to the objective lens 132.

Note that, each injection port 141C is opened toward the objective lens 132, has a substantially oval shape extending in the intersecting direction, and has an arc shape. Further, an opening width W3 of each injection port 141C in the protruding direction (Z direction) is constant in the intersecting direction.

Further, in each of the injection ports 141C, a dimension H3 (see a dashed arrow in Fig. 9) in the protruding direction from one point (see a point P in Fig. 9) of an edge on the distal end surface 131 side to an edge on an opposing side increases toward one end portion 141b on the other injection port 141C side.

In the example of Fig. 9, a case where the one point (point P) is the position closest to the distal end surface 131 has been described as an example, but the present invention is not limited thereto.

The two injection ports 141C are arranged on the same arc. That is, a line (see a dashed line in Fig. 9) connecting an intermediate position between a side edge on the lid portion 148 side and a side edge on the distal end surface 131 side in each of the injection ports 141C forms one arc centered on an outside of the injection port 141C. Note that, such an arc may have the same radius of curvature as the objective lens 132.

Air or water injected from the two injection ports 141C spreads in the intersecting direction and partially merges. Due to the Coanda effect in which a fluid flowing near a wall surface is attracted to the wall surface by the effect of fluid viscosity, air or water injected from the injection ports 141C merges with each other and then flows along a surface (curved surface) of the objective lens 132.

With the above configuration, the air and water supply nozzle 140 of the endoscope 10 according to the fourth embodiment can inject air or water according to a curved surface shape of the objective lens 132 which is a convex lens.

That is, in the endoscope 10 according to the fourth embodiment, as described above, each injection port 141C is formed such that the dimension H3 increases toward the one end portion 141b on the other injection port 141C side.

Therefore, when the two injection ports 141C inject air or water, the central portion in the intersecting direction of the injected air or water is higher than other portions, in the protruding direction (Z direction) from the distal end surface 131 (or XY plane). That is, air or water is injected according to a curved surface shape of a surface of the objective lens 132 which is a convex lens. Therefore, the objective lens 132 can be cleaned sufficiently and efficiently.

Parts similar to those in the first embodiment are denoted by the same reference numerals, and detailed descriptions thereof are omitted.

### (Modification)

Fig. 10 is a schematic view schematically illustrating a modification of the air and water supply nozzle 140 of Fig. 9. In Fig. 10, a distal end surface 131 of a distal end portion 13 is indicated by a one-dot chain line for convenience of description. Further, in Fig. 10, a Z direction is indicated by an outlined arrow, and a plane (XY plane) perpendicular to the Z direction is indicated by a two-dot chain line.

An injection portion 149 in an air and water supply nozzle 140 according to the present modification has two injection ports 141D through which air or water is injected, and is a portion including the two injection ports 141D and an interval between the two injection ports 141D.

When injecting air or water, the injection portion 149 injects the air or water so that a central portion in an intersecting direction of the injected air or water is higher than other portions, in a protruding direction (Z direction) of the air and water supply nozzle 140 (or the objective lens 132) from the distal end surface 131 (or XY plane) .

The two injection ports 141D are juxtaposed in the intersecting direction. In addition, a tangent line T (see a solid line T in Fig. 10) in contact with each of the two injection ports 141D on the distal end side of the air and water supply nozzle 140 is convexly curved in the protruding direction (Z direction), similarly to the objective lens 132.

Note that, each injection port 141D is open toward the objective lens 132 and has a substantially arc shape. The injection port 141D is different in shape from the injection port 141C. An opening width W4 of each of the injection ports 141D in the protruding direction increases toward one end portion 141b on the other injection port 141D side.

Further, in each of the injection ports 141D, a dimension H4 (see a dashed arrow in Fig. 10) in the protruding direction from one point (see a point P in Fig. 10) of a side edge of the distal end surface 131 to an edge on an opposing side increases toward one end portion 141b on the other injection port 141D side.

In the example of Fig. 10, a case where the one point (point P) is the position closest to the distal end surface 131 has been described as an example, but the present invention is not limited thereto.

Then, the two injection ports 141D may be arranged on the same arc, and such an arc may have the same radius of curvature as the objective lens 132.

With the above configuration, the air and water supply nozzle 140 according to the present modification can also inject air or water according to a curved surface shape of the objective lens 132 which is a convex lens, similarly to the air and water supply nozzle 140 of Fig. 9, and the same effect is obtained.

Furthermore, in the air and water supply nozzle 140 according to the present modification, as described above, the opening width W4 of each injection port 141D increases toward the one end portion 141b on the another injection port 141D side, and thus, an injection amount increases at the central portion of the injected air or water. Therefore, the central portion of the objective lens 132 can be intensively cleaned, and a better captured image can be acquired.

Parts similar to those in the first embodiment are denoted by the same reference numerals, and detailed descriptions thereof are omitted.

### (Fifth Embodiment)

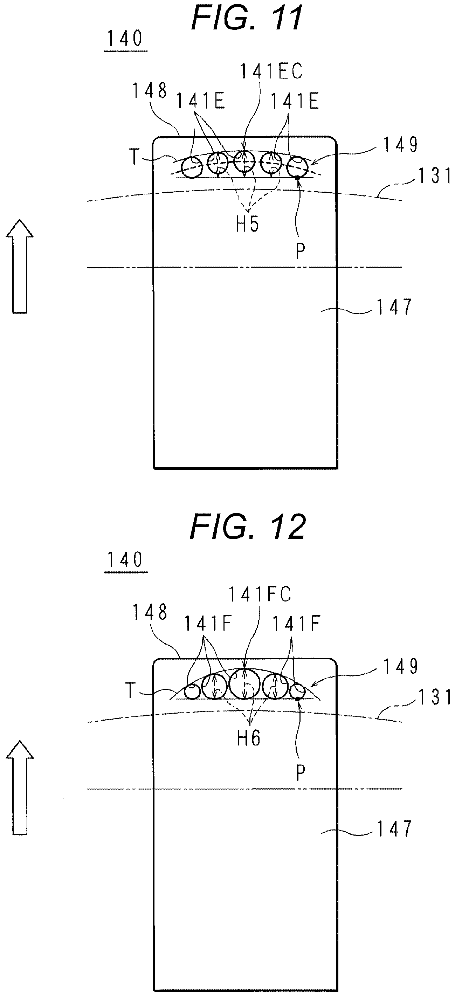

Fig. 11 is a schematic view schematically illustrating an air and water supply nozzle 140 of an endoscope 10 according to a fifth embodiment of the present invention. In Fig. 11, a distal end surface 131 of a distal end portion 13 is indicated by a one-dot chain line for convenience of description. Further, in Fig. 11, a Z direction is indicated by an outlined arrow, and a plane (XY plane) perpendicular to the Z direction is indicated by a two-dot chain line.

Similarly to the first embodiment, the air and water supply nozzle 140 includes a cylindrical portion 147, an injection portion 149, a lid portion 148, a first guide wall 144, and a second guide wall 145, and the lid portion 148, the injection portion 149, the cylindrical portion 147, the first guide wall 144, and the second guide wall 145 are integrally formed.

The injection portion 149 has three or more injection ports 141E through which air or water is injected, and is a portion including the three or more injection ports 141E and an interval between the injection ports 141E.

When injecting air or water, the injection portion 149 injects the air or water so that a central portion in an intersecting direction of the injected air or water is higher than other portions, in a protruding direction (Z direction) of the air and water supply nozzle 140 (or an objective lens 132) from the distal end surface 131 (or XY plane).

Further, the injection portion 149 has a configuration similar to that of the first embodiment, but is different in that the three or more injection ports 141E are included, and a shape of the injection port 141E is different. Hereinafter, for convenience, a case where the number of injection ports 141E is five will be described as an example.

The five injection ports 141E are juxtaposed in the intersecting direction. In addition, a tangent line T (see a solid line T in Fig. 11) in contact with each of the five injection ports 141E on the distal end side of the air and water supply nozzle 140 is convexly curved in the protruding direction (Z direction), similarly to the objective lens 132.

Furthermore, each injection port 141E is open toward the objective lens 132 and has a circular shape. The injection ports 141E each have the same size.

In addition, the five injection ports 141E are provided such that a dimension H5 (see a dashed arrow in Fig. 11) increases as the injection port 141E is closer to the center in a juxtaposition direction (intersecting direction). The dimension H5 is a dimension in the protruding direction (Z direction) from one point (see a point P in Fig. 11) of an edge on the distal end surface 131 side of any one of the injection ports 141E to an edge on an opposing side of each of the injection ports 141E, that is, to an edge on the distal end side of the air and water supply nozzle 140. In the example of Fig. 11, the dimension H5 of a central injection port 141EC among the five injection ports 141E is the largest.

In the example of Fig. 11, a case where the one point (point P) is the position closest to the distal end surface 131 has been described as an example, but the present invention is not limited thereto.

The five injection ports 141E are arranged on the same arc. That is, a line (see a dashed line in Fig. 11) connecting centers of the individual injection ports 141E forms one arc centered on an outside of the injection port 141E. Note that, such an arc may have the same radius of curvature as the objective lens 132.

Note that, due to the Coanda effect described above, air or water injected from the injection ports 141E merges with each other and then flows along a surface (curved surface) of the objective lens 132.

With the above configuration, the air and water supply nozzle 140 of the endoscope 10 according to the fifth embodiment can inject air or water according to a curved surface shape of the objective lens 132 which is a convex lens.

That is, in the endoscope 10 according to the fifth embodiment, as described above, the injection port 141E is provided such that the injection port 141E closer to the center in the juxtaposition direction has a larger dimension H5.

Therefore, the central portion in the intersecting direction of the injected air or water is higher than other portions, in the protruding direction (Z direction) from the distal end surface 131 (or XY plane). That is, air or water is injected according to a curved surface shape of a surface of the objective lens 132 which is a convex lens. Therefore, the objective lens 132 can be cleaned sufficiently and efficiently.

In the above description, a case where the number of the injection ports 141E is five has been described as an example, but the invention is not limited thereto, and the number of the injection ports 141E may be three or four, or may be six or more.

Parts similar to those in the first embodiment are denoted by the same reference numerals, and detailed descriptions thereof are omitted.

### (Modification)

Fig. 12 is a schematic view schematically illustrating a modification of the air and water supply nozzle 140 of Fig. 11. In Fig. 12, a distal end surface 131 of a distal end portion 13 is indicated by a one-dot chain line for convenience of description. Further, in Fig. 12, a Z direction is indicated by an outlined arrow, and a plane (XY plane) perpendicular to the Z direction is indicated by a two-dot chain line.

An injection portion 149 in an air and water supply nozzle 140 according to the present modification has five injection ports 141F through which air or water is injected, and is a portion including the five injection ports 141F and an interval between the injection ports 141F.

When injecting air or water, the injection portion 149 injects the air or water so that a central portion in an intersecting direction of the injected air or water is higher than other portions, in a protruding direction (Z direction) of the air and water supply nozzle 140 (or the objective lens 132) from the distal end surface 131 (or XY plane).

The five injection ports 141F are juxtaposed in the intersecting direction. In addition, a tangent line T (see a solid line T in Fig. 12) in contact with each of the five injection ports 141F on the distal end side of the air and water supply nozzle 140 is convexly curved in the protruding direction (Z direction), similarly to the objective lens 132. Furthermore, each injection port 141F is open toward the objective lens 132 and has a circular shape.

Further, the five injection ports 141F have different sizes. The five injection ports 141F are configured such that the injection port 141F closer to the center has a larger size. That is, a radius of each of the five injection ports 141F is larger as the injection port 141F is closer to the center, and in the example of Fig. 12, a size of a central injection port 141FC among the five injection ports 141F is the largest.

Furthermore, the five injection ports 141F are provided such that a dimension H6 (see a dashed arrow in Fig. 12) increases as the injection port 141F is closer to the center in the juxtaposition direction (intersecting direction). The dimension H6 is a dimension in the protruding direction (Z direction) from one point (see a point P in Fig. 12) of an edge on the distal end surface 131 side of any one of the injection ports 141F to an edge on an opposing side of each of the injection ports 141F, that is, to an edge on the distal end side of the air and water supply nozzle 140. Note that, in the example of Fig. 12, the dimension H6 of the central injection port 141FC among the five injection ports 141F is the largest.

In the example of Fig. 12, a case where the one point (point P) is the position closest to the distal end surface 131 has been described as an example, but the present invention is not limited thereto.

Note that the five injection ports 141F may be arranged on the same arc, and such an arc may have the same radius of curvature as the objective lens 132.

With the above configuration, the air and water supply nozzle 140 according to the present modification can also inject air or water according to a curved surface shape of the objective lens 132 which is a convex lens, similarly to the air and water supply nozzle 140 of Fig. 11, and the same effect is obtained.

Furthermore, in the air and water supply nozzle 140 according to the present modification, as described above, since the radius of the five injection ports 141F is larger as the injection port 141F is closer to the center, an injection amount is larger in the central portion of the injected air or water. Therefore, the central portion of the objective lens 132 can be intensively cleaned, and a better captured image can be acquired.

Parts similar to those in the first embodiment are denoted by the same reference numerals, and detailed descriptions thereof are omitted.

### (Sixth embodiment)

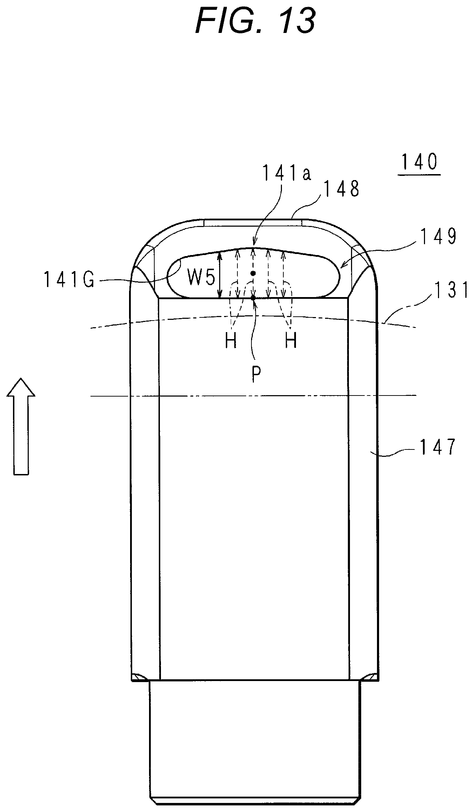

Fig. 13 is a schematic view schematically illustrating an air and water supply nozzle 140 of an endoscope 10 according to a sixth embodiment of the present invention. In Fig. 13, a distal end surface 131 of a distal end portion 13 is indicated by a one-dot chain line for convenience of description. Further, in Fig. 13, a Z direction is indicated by an outlined arrow, and a plane (XY plane) perpendicular to the Z direction is indicated by a two-dot chain line.

Similarly to the first embodiment, the air and water supply nozzle 140 includes a cylindrical portion 147, an injection portion 149, a lid portion 148, a first guide wall 144, and a second guide wall 145, and the lid portion 148, the injection portion 149, the cylindrical portion 147, the first guide wall 144, and the second guide wall 145 are integrally formed.

The injection portion 149 is a portion including an injection port 141G through which air or water is injected. When injecting air or water, the injection portion 149 injects the air or water so that a central portion in an intersecting direction of the injected air or water is higher than other portions, in a protruding direction (Z direction) of the air and water supply nozzle 140 (or an objective lens 132) from the distal end surface 131 (or XY plane).

The injection portion 149 of the sixth embodiment has a configuration similar to that of the first embodiment, but a shape of the injection port 141G is different.

The injection port 141G is opened toward the objective lens 132, and has a substantially oval shape with the intersecting direction as a long axis direction. In the injection port 141G, similarly to the objective lens 132, the one edge has a convex shape in the protruding direction, and the another edge is parallel to the XY plane.

In addition, an opening width W5 of the injection port 141G increases toward a central portion 141a in the intersecting direction. Here, the opening width W5 indicates a dimension between the one edge and the another edge of the injection port 141G.

Further, both end portions of the injection port 141G in the intersecting direction have a semicircular arc shape. That is, in the injection port 141G, edges of both end portions are curved in a semicircular shape and are not angular.

With the above configuration, in the endoscope 10 according to the sixth embodiment, when air or water is injected, the central portion in the intersecting direction of the injected air or water is higher than other portions, in the protruding direction (Z direction) from the distal end surface 131 (or XY plane). That is, air or water is injected according to a curved surface shape of a surface of the objective lens 132 which is a convex lens. Therefore, the objective lens 132 can be cleaned sufficiently and efficiently.

Further, in the endoscope 10 according to the sixth embodiment, as described above, both end portions of the injection port 141G have a semicircular arc shape. Therefore, it is possible to suppress an occurrence of an injection pressure loss due to an occurrence of turbulence in a fluid at both end portions of the injection port 141G, and to efficiently inject the fluid to the objective lens 132.

Parts similar to those in the first embodiment are denoted by the same reference numerals, and detailed descriptions thereof are omitted.

Technical features (constitutional requirements) that have been described in the first to sixth embodiments can be combined with one another, and a new technical feature can be formed with the combination.

It should be noted that the embodiments disclosed herein are illustrative in all respects and are not restrictive. The scope of the present invention is indicated by the scope of claims, not the above-described meaning, and is intended to include all modifications within the meaning and scope equivalent to the claims.

### Reference Signs List

10 Endoscope
14 Insertion portion
131, 131A Distal end surface
132 Objective lens
140 Air and water supply nozzle
141, 141A to 141F Injection port
141a Central portion
141b One end portion
149 Injection portion
H, H1 to H6 Dimension
P One point
T Tangential line
W, W1 to W5 Opening width

## Claims

1. An endoscope comprising: a convex lens (132) provided protrusively on a distal end surface (131) of an insertion portion (14); and a nozzle (140) provided protrusively on the distal end surface (131) and configured to inject a fluid to the convex lens (132), wherein
the nozzle (140) has one or a plurality of injection ports (141),
in a case where the nozzle (140) has one injection port (141),
the injection port (141) extends in an intersecting direction intersecting with a protruding direction of the convex lens (132), and
at least one edge close to a distal end of the nozzle (140) among two edges of the injection port (141) facing each other in the protruding direction has a convex shape in the projecting direction, and
in a case where the nozzle (140) has a plurality of injection ports (141),
the plurality of injection ports (141) are formed along the intersecting direction, and
a tangent line in contact with each of the plurality of injection ports (141) on a distal end side of the nozzle (140) is convexly curved in the protruding direction.

2. The endoscope (10) according to claim 1, wherein
in a case where the nozzle (140) has one injection port (141),
a dimension (H) of the injection port (141) between the one edge and one point of another edge is large at a central portion (141a) in the intersecting direction, as compared to other portion.

3. The endoscope (10) according to claim 2, wherein an opening width (W) of the injection port (141) in the protruding direction increases toward the central portion (141a).

4. The endoscope (10) according to claim 2, wherein the another edge of the injection port (141) has a convex shape in the protruding direction.

5. The endoscope (10) according to claim 2, wherein the injection port (141A) has an inverted V-shape, and an opening width (W1) in the protruding direction is constant.

6. The endoscope (10) according to claim 2, wherein the injection port (141B) has an arc shape, and an opening width (W2) in the protruding direction is constant.

7. The endoscope (10) according to claim 1, wherein
the nozzle (140) has two injection ports (141C) formed along the intersecting direction, and
in each of the injection ports (141C), a dimension (H3) in the protruding direction from one point of an edge on the distal end surface (131) side to an edge on an opposing side increases toward one end portion (141b) close to another one of the injection ports (141C).

8. The endoscope (10) according to claim 7, wherein the two injection ports (141C) are arranged on a same arc.

9. The endoscope (10) according to claim 7 or 8, wherein an opening width (W4) of each injection port (141D) in the protruding direction increases toward the one end portion (141b).

10. The endoscope (10) according to claim 1, wherein
the nozzle (140) has three or more injection ports (141E) formed along the intersecting direction, and
a dimension (H5) in the protruding direction from one point of an edge on the distal end surface (131) side of any injection port (141E) to an edge on a distal end side of the nozzle (140) of each injection port (141E) increases toward a center.

11. The endoscope (10) according to claim 10, wherein the three or more injection ports (141F) are arranged on a same arc.

12. The endoscope (10) according to claim 10 or 11, wherein each of the three or more injection ports (141E) is larger in size as being closer to the center.
